# EUROPEAN PATENT APPLICATION

(11) **EP 4 410 336 A1**
(43) Date of publication of application: **07.08.2024**
(21) Application number: 23154701.9
(22) Date of filing: 02.02.2023
(51) Int. Cl.: A61M 5/32

(54) **NEEDLE SAFETY DEVICE**

(71) Applicant: Sensile Medical AG, 4600 Olten (CH)
(72) Inventor: FORSTER, Richard, 92269 Fensterbach (DE); GORSHÖFER, Andreas, 93149 Nittenau (DE); JAKOB, Michael, 93051 Regensburg (DE); PFRANG, Jürgen, 93183 Kallmünz (DE)
(74) Representative: Bardehle Pagenberg Partnerschaft mbB Patentanwälte Rechtsanwälte

(57) **Abstract**

The present disclosure relates to a needle safety device (1) comprising an elongated hub portion (10) having a longitudinal axis (11). The hub portion (10) comprises a cannula (12). Further, the device (1) comprises a tubular protection element (20) arranged to be movable along the longitudinal axis (11) relative to the hub portion (10), wherein the protection element (20) comprises a contact surface (21) for establishing pressure contact with an injection area. Moreover, the device (1) comprises a tubular separable element (30) comprising at least two locking parts (31, 32). Furthermore, the device (1) comprises a spring element (40) which biases the protection element (20) such that the contact surface (21) is further spaced apart from the hub portion (10) along the longitudinal axis (11) than the tip (13) of the cannula (12). The protection element (20) is configured to move into an injection position, wherein the separable element (30) is removed from the protection element (20) and thereby separates into the locking parts (31, 32) when the contact surface (21) is released from contact with the injection area. The separated locking parts (31, 32) block the protection element (20) from returning into the injection position when the protection element (20) has moved to a final position.

## Description

### 1. Technical field

The present disclosure relates to a needle safety device for a medical injection device and a medical injection device comprising the needle safety device.

### 2. Prior art

For some medical injection devices, such as auto-injectors or injection pens, it is often necessary to ensure that no intended and/or unintended reuse of the medical injection device can take place. The reasons for this can be manifold. For example, the medical injection device in question may be intended for only one injection procedure, such as an auto-injector that injects an emergency medication like adrenaline in the event of an allergic shock. In addition, it is understood that hygiene-related reasons are also conceivable. In this regard, especially cross-contamination after injection is to be avoided, where a person is injured and contaminated by a used needle.

Furthermore, for some medical injection devices, especially those being operated also by non-medical personnel, such as again auto-injectors or injection pens, it must be ensured that no unintentional contact with the cannula can take place. For example, to prevent injuries, contamination of the user, and/or contamination of the cannula.

Various solutions, in particular needle safety devices, are known from the prior art to prevent the reuse of a medical injection device and to prevent accidental contact with a cannula of the medical injection device. In general, known needle safety devices work on the principle that a sleeve is provided which is arranged around the needle in such a way that unintentional contact with this same needle is prevented. Further, the sleeve is configured to be displaced once by contact with an injection area such that injection can take place, whereas displacement of the sleeve again after injection is prevented, exemplarily by a locking means. However, the specific solutions of the prior art, respectively the existing needle safety devices, have several disadvantages, some of which are presented below.

First, some existing needle safety devices for medical injection devices cause the injection area to experience torsion. For example, because the sleeve surrounding the needle undergoes rotation relative to the medical injection device during injection, e.g. to effect locking. This rotation is regularly perceived by patients as unpleasant and, in the worst case, leads to the patient interrupting the injection.

Second, some needle safety devices allow a locking means, which is intended to fix the sleeve after injection in such a way that the cannula is permanently protected from contact, to be released again. One reason for this can be that the movement that led to the locking of the sleeve can be reversed manually, for example by turning the sleeve back to the unlocked initial state. Another reason can be that the locking means itself may be manually unlocked again. For example, by releasing a locking arm or, as in the embodiment shown in US 9,907,916 B2, by moving the pin out of the final position in which the pin is supposed to be retained.

Thus, it is an object of the present disclosure to provide a needle safety device for a medical injection device and a medical injection device that overcome the aforementioned drawbacks at least partially.

### 3. Summary of the invention

This object is achieved, at least partly, by a needle safety device for a medical injection device and a medical injection device, as defined in the independent claims. Further aspects of the present disclosure are defined in the dependent claims.

In particular, the object is achieved by a needle safety device for a medical injection device. The needle safety device may serve to prevent the reuse of a medical injection device and to prevent accidental contact with a piercing means such as a cannula, wherein optionally the needle safety device may provide additional functionalities. The medical injection device may be an auto-injector, an injection pen, and/or a syringe. However, preferably the medical injection device is an auto-injector. This is as auto-injectors particularly rely thereon that reuse is prevented and that accidental contact with the piercing means is avoided. Exemplarily since auto-injectors are regularly operated by non-medical personnel, e.g. in emergency situations.

The needle safety device comprises an elongated hub portion having a longitudinal axis, wherein the hub portion comprises a cannula extending from the hub portion substantially along the longitudinal axis. The longitudinal axis of the hub portion may also be a longitudinal center axis of the hub portion. Particularly, if the hub portion has a substantially cylindrical shape. The cannula may be also referred to as hollow needle or injection needle. Further, the cannula may be attached directly or indirectly to the hub portion. Said cannula may be part of a needle array. Moreover, it is understood that the cannula may be designed to be movable relative to the hub portion, for example to be able to realize a penetration of a liquid reservoir within the medical injection device.

Further, the needle safety device comprises a tubular protection element being arranged to be movable along the longitudinal axis relative to the hub portion, wherein the protection element comprises a contact surface for establishing pressure contact with an injection area. In an initial position of the needle safety device the contact surface is further spaced apart from the hub portion along the longitudinal axis than the tip of the cannula. The tubular protection element allows that the tip of the cannula can be protected against unintentional contact. Particularly, in the initial position, i.e., before starting the injection process, the protection element can prevent contact with the tip of the cannula. Exemplarily, when approaching the tip of the cannula with a human finger, the finger first abuts the contact surface instead of immediately contacting the tip of the cannula. It is understood that said protection element may surround the cannula.

Moreover, the needle safety device comprises a tubular separable element being removably arranged in the protection element in the initial position such that the separable element is movable along the longitudinal axis together with the protection element, wherein the separable element comprises at least two locking parts. Exemplarily, the tubular separable element may be removably attached to the protection element in the initial position. Thereby the tubular separable element may be attached directly or indirectly. Exemplarily, an inside surface of the tubular protection element may releasably engage with an outside surface of the tubular separable element, e.g., by means of a form-fit connection.

Furthermore, the needle safety device comprises a spring element which biases the protection element in a distal direction relative to the hub portion such that the contact surface is further spaced apart from the hub portion along the longitudinal axis than the tip of the cannula when the contact surface is not in pressure contact with the injection area. By means of the described biasing of the protection element an accidental contact with the tip of the cannula can be prevented. Particularly, when the contact surface is not in pressure contact with the injection area, i.e., when no injection is taking place. The spring element may be a coil spring. However, it is understood that different spring elements are conceivable.

The protection element is configured to move into an injection position when the contact surface is brought in pressure contact with the injection area, wherein in the injection position the tip of the cannula is further spaced apart from the hub portion along the longitudinal axis than the contact surface, wherein the separable element is removed from the protection element and thereby separates into the at least two locking parts when the contact surface is released from pressure contact with the injection area, wherein the separated locking parts block the protection element from returning into the injection position when the protection element has moved to a final position, wherein in the final position the contact surface is further spaced apart from the hub portion along the longitudinal axis than the tip of the cannula. By the tip of the cannula in the injection position being further spaced apart from the hub portion along the longitudinal axis than the contact surface, the tip of the cannula can be inserted into the injection area. Further, the separable element may be detached from the protection element, e.g. by being retained by the hub portion, while the spring element biases the protection element in the distal direction relative to the hub portion. Furthermore, the separable element may exemplarily separate into the at least two locking parts due to a pretension in the separable element which is released after the separable element being removed from the protection element. Moreover, the separable element may exemplarily separate into the at least two locking parts due to respective provisions on the separable element and/or the hub portion. Exemplarily, the separated locking parts may block the protection element from returning into the injection position in that the separation causes the locking parts to be arranged in such a way that the mobility of the protection element in the direction of the longitudinal axis is restricted. The separated locking parts blocking the protection element from returning into the injection position allows that the needle safety device serves to prevent the reuse of a medical injection device.

The described needle safety device has several advantages over the prior art, wherein two of them are set out below in detail.

First, as the instant needle safety device does not rely on a guide pin which slides through a guide track of a rotating sleeve, the needle safety device allows for configurations which do not cause the injection area to experience torsion. This can make the injection process more comfortable for a patient.

Second, the tubular separable element makes it more difficult to transfer the needle safety device back to the initial position intentionally as well as unintentionally, especially in comparison to devices where a guide pin engages with a guide track. This is as the separated locking parts would need to be recombined and again arranged in the protection element. This has proven to be exceedingly difficult and results in a high level of security of the needle safety device against reuse.

It is understood that the described advantages may apply also for the following in different expressions.

Optionally, in the initial position the at least two locking parts are not materially bonded to each other. Exemplarily, the locking parts may abut against each other. Further exemplarily, in the initial position the locking parts may be pretensioned against each other within the protection element. Hence, the separable element may separate into the at least two locking parts due to said pretension being released after removing the separable element from the protection element. By not being materially bonded to each other the separation of the locking parts may be conducted more reliable.

In the initial position the separable element may be at least partially enclosed by the protection element such that the at least two locking parts are hold together and abut against each other. This configuration allows that an undesired removal of the separable element from the protection element can be avoided, particularly due to the separable element being at least partially enclosed by the protection element. Further, the configuration allows for a facilitated separation of the separable element into the locking parts after removing it from the protection element.

In the initial position the at least two locking parts may be integrally formed with each other. Here "integrally formed" may refer to the aspect that at least one position it is not possible to determine a material boundary between the at least two locking parts. It has been found that by forming the at least two locking parts integrally with each other, a simplified assembly of the needle safety device can take place. In addition, undesired separation can be more easily avoided.

Further, the contact surface may have an opening through which the tip of the cannula passes when the protection element is transferred into the injection position. Thereby the opening may have a diameter which avoids the insertion of a human finger into the protection element.

Moreover, the protection element may be configured such that it substantially does not rotate relative to the hub portion. Thus, the injection area can be particularly prevented from experiencing torsion. This makes the injection process more comfortable for a patient. Exemplarily, the protection element may directly engage with the hub portion. Alternatively, an intermediate element, such as a housing, which is fixedly attached to the hub portion may ensure that protection element cannot rotate relative to the hub portion.

The protection element and the separable element may be configured such that they allow for a rotation relative to each other, wherein optionally the separable element is in rotatable sliding engagement with the protection element. This rotation can be used, for example, to reliably remove the separable element from the protection element.

The hub portion and the separable element may each comprise retention means being configured to engage with each other such that the separable element is retained in a position relative to the hub portion when the protection element moves to the final position. It is understood that the protection element moves to the final position from the injection position. By being retained in position relative to the hub portion when the protection element moves from the injection position to the final position the separable element may be reliably removed from the protection element. For example, the retention means may each comprise abutment surfaces configured to be engaged with each other.

Optionally, the hub portion and/or the separable element comprise rotating means which rotate the separable element relative to the hub portion when the protection element with the separable element arranged therein is moved from the initial position into the injection position, wherein rotating the separable element relative to the hub portion leads to the retention means to engage with each other. Preferably the rotating means are configured to rotate the separable element relative to the hub portion around the longitudinal axis. By the feature, wherein rotating the separable element relative to the hub portion leads to the retention means to engage with each other it can on one hand be avoided that an unintentional removal of the separable element from the protection element occurs and on the other hand it is particularly difficult to remove the needle safety device from the final position into the initial position. Accordingly, the overall safety of the needle safety device can be improved.

Furthermore, in the initial position the separable element may be removably arranged in the protection element by means of a form-fit connection, wherein optionally the protection element comprises a protrusion and the separable element comprises a groove, wherein in the initial position the protrusion and the groove are in engagement. Exemplarily, the protrusion may be provided on the inside of the protection element and the groove may be provided on the outside of the separable element. It has been shown that form-fit connections allow a reliable connection on the one hand and ensure easy release on the other. Moreover, such connections allow in particular to provide the described rotatability between separable element and protection element.

The at least two locking parts may move radially outward as seen from the longitudinal axis and thereby separate from each other when the separable element is removed from the protection element, wherein optionally the hub portion and/or the separable element have separating means for moving the at least two locking parts radially outward. Said separation may lead to the locking parts to be positioned such that they block the protection element from returning into the injection position when the protection element has moved to a final position. Exemplarily, said separating means may be ramp shaped elements which urge the at least two locking parts radially outward, e.g., due to gravity.

Optionally, the hub portion comprises a first abutment surface and the protection element comprises a second abutment surface, wherein if in the final position a pressure is applied onto the contact surface such that at least one of the separated locking parts abuts the first abutment surface and the second abutment surface then the protection element is blocked by said separated locking part from returning into the injection position. Hence, the reuse of the needle safety device is prevented.

The spring element may abut the hub portion and the protection element. This configuration has proven to be advantageous in that it prevents the spring element from affecting the rotation of the separable element.

Further, the above object is achieved by a medical injection device comprising the needle safety device as described above. Since the medical injection device comprises the needle safety device as described above, it is understood that the features and/or advantages described with respect to the needle safety device may also apply for the medical injection device.

The medical injection device may comprise a housing which engages with the protection element such that the protection element substantially does not rotate relative to the hub portion.

### 4. Brief description of the accompanying figures

In the following, the accompanying figures are briefly described:
Fig. 1 shows a needle safety device according to the present invention in perspective view prior to injection;
Fig. 2 shows the needle safety device in cut view prior to injection;
Fig. 3 shows the needle safety device in cut view during injection;
Fig. 4 shows the needle safety device in cut view after injection;
Fig. 5 shows the needle safety device in cut view after injection;
Fig. 6 shows the needle safety device in cut view after injection when an attempt is made to actuate the device again;
Fig. 7 shows the tubular separable element of the needle safety device in perspective view;
Fig. 8 shows the needle safety device according to the present invention in perspective view after injection, and
Fig. 9 shows a medical injection device according to the present disclosure after injection.

### 5. Detailed description of the figures

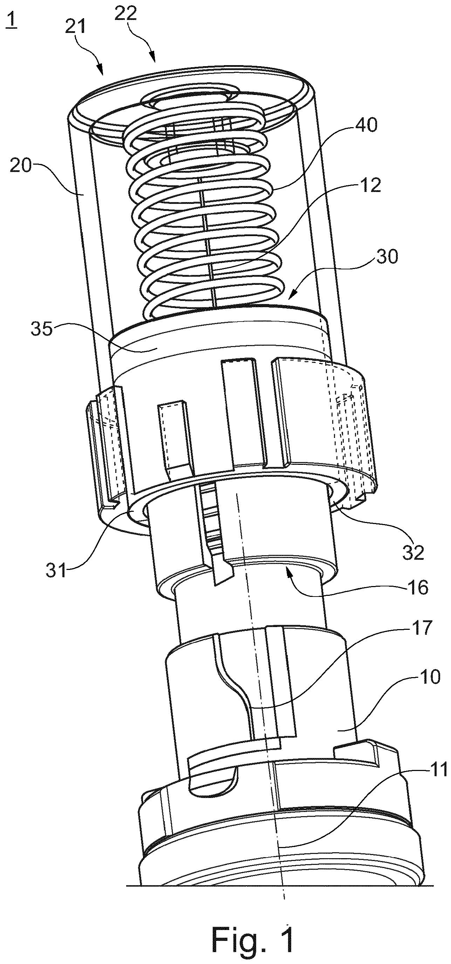

**Figs. 1 to 9** show the needle safety device 1 according to the present disclosure or parts thereof. The needle safety device 1 comprises an elongated hub portion 10 having a longitudinal center axis 11.

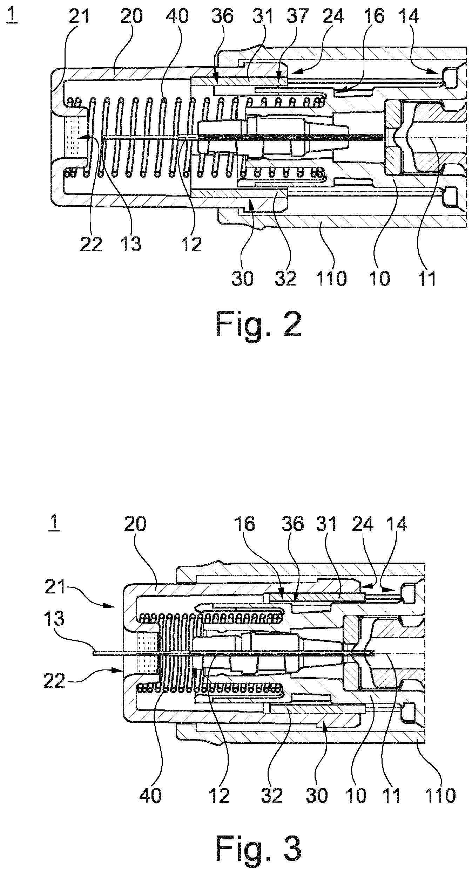

As e.g., shown in detail in **Fig. 2****,** the hub portion 10 comprises a cannula 12 extending from the hub portion 10 substantially along the longitudinal axis 11. Particularly, the cannula 12 is attached indirectly to the hub portion via a carrier element which is fixedly attached to the cannula 12. Moreover, the cannula is designed to be movable relative to the hub portion, to realize a penetration of a liquid reservoir within the medical injection device 100.

Further, the hub portion 10 comprises a tubular protection element 20 being arranged to be movable along the longitudinal axis 11 relative to the hub portion 10. Said protection element 20 comprises a contact surface 21 for establishing pressure contact with an injection area, wherein in an initial position of the needle safety device 1, which is e.g. shown in **Figs. 1** **and** **2****,** the contact surface 21 is further spaced apart from the hub portion 10 along the longitudinal axis 11 than the tip 13 of the cannula 12. The contact surface 21 has an opening 22 through which the tip 13 of the cannula 12 passes when the protection element 20 is transferred into the injection position (s. **Fig. 3**).

Moreover, the hub portion 10 comprises a tubular separable element 30 being removably arranged in the protection element 20 in the initial position such that the separable element 30 is movable along the longitudinal axis 11 together with the protection element 20, wherein the separable element 30 comprises at least two locking parts 31, 32.

Furthermore, the hub portion 10 comprises a spring element 40 which biases the protection element 20 in a distal direction relative to the hub portion 10 such that the contact surface 21 is further spaced apart from the hub portion 10 along the longitudinal axis 11 than the tip 13 of the cannula 12 when the contact surface 21 is not in pressure contact with the injection area (s. e.g., **Figs. 1** **and** **2**)**.** The spring element 40 abuts the hub portion 10 and the protection element 20.

The protection element 20 is configured to move into an injection position, which is e.g. shown in **Fig. 3****,** when the contact surface 21 is brought in pressure contact with the injection area, wherein, as depicted, in the injection position the tip 13 of the cannula 12 is further spaced apart from the hub portion 10 along the longitudinal axis 11 than the contact surface **21.**

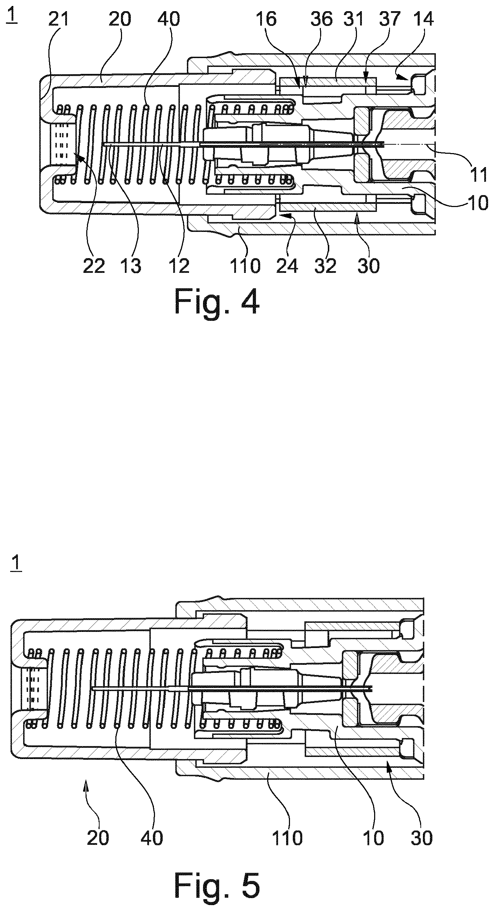

Further, as illustrated by **Figs. 3** **and** **4****,** the separable element 30 is removed from the protection element 20 and thereby separates into the at least two locking parts 31, 32 when the contact surface 21 is released from pressure contact with the injection area. Particularly, the at least two locking parts 31, 32 move radially outward as seen from the longitudinal axis 11 and thereby separate from each other when the separable element 30 is removed from the protection element 20.

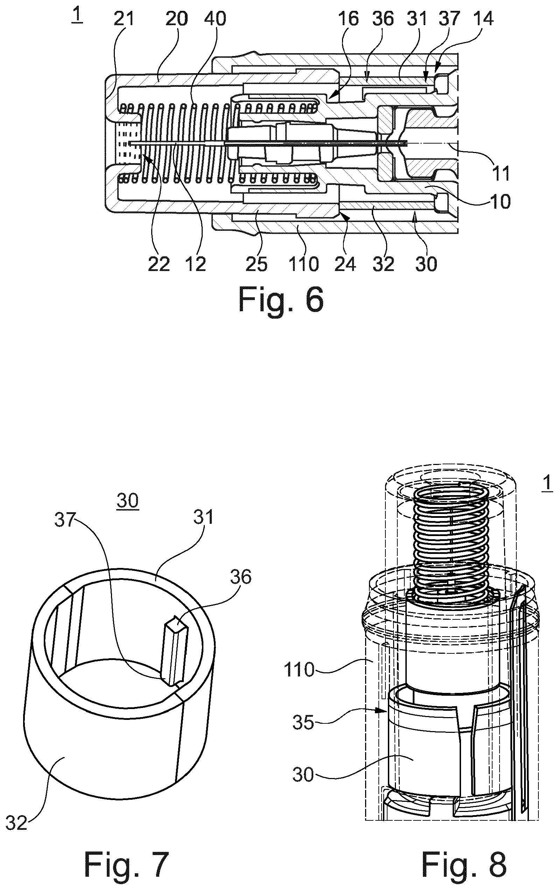

Thereby, as shown in **Fig. 6****,** the separated locking parts 31, 32 block the protection element 20 from returning into the injection position when the protection element 20 has moved to a final position.

In the final position (s. **Figs. 5** **and** **8**) the contact surface 21 is further spaced apart from the hub portion 10 along the longitudinal axis 11 than the tip 13 of the cannula 12.

As illustrated by **Fig. 7****,** in the initial position the at least two locking parts 31, 32 are not materially bonded to each other. Rather, as the skilled person understands from **Figs. 1** **and** **2****,** in the initial position the separable element 30 is enclosed by the protection element 20 such that the at least two locking parts 31, 32 are hold together and abut against each other.

The protection element 20 is configured such that it substantially does not rotate relative to the hub portion 10. Exemplarily, in **Fig. 8** protrusions of the protection element 20 are in engagement with guiding means of the housing 110 of the medical injection device such that the protection element 20 substantially does not rotate relative to the hub portion 10 which is attached to the housing 110.

Further, the protection element 20 and the separable element 30 are configured such that they allow for a rotation relative to each other, wherein the separable element 30 is in rotatable sliding engagement with the protection element 20. Particularly, in the initial position the separable element 30 is removably arranged in the protection element 20 by means of a form-fit connection, wherein the protection element 20 comprises a protrusion 25 and the separable element 30 comprises a groove 35, wherein in the initial position the protrusion 25 and the groove 35 are in engagement. This engagement allows that the protection element 20 and the separable element 30 can be rotated relative to each other.

Moreover, the hub portion 10 and the separable element 30 each comprise retention means 16, 36 being configured to engage with each other such that the separable element 30 is retained in a position relative to the hub portion 10 when the protection element 20 moves to the final position (s. **Fig. 3** **and** **4**).

It is understood by the skilled person that in **Fig. 4** the state is shown right after the separable element 30 has been separated from the protection element 20, whereas in **Fig. 5** the state is shown, when the protection element 20 has been fully returned by the spring element 40 and the locking parts 31, 32 have fallen down within the housing 110 due to gravity.

Additionally, the hub portion 10 and the separable element 30 comprise rotating means 17, 37 which rotate the separable element 30 relative to the hub portion 10 when the protection element 20 with the separable element 30 arranged therein is moved from the initial position into the injection position, wherein rotating the separable element 30 relative to the hub portion 10 leads to the retention means 16, 36 to engage with each other (s. **Fig. 2 and 3**).

Particularly, the skilled person understands from **Figs. 1 to 4** that the retention means 16 of the hub portion 10 is a circumferential abutment surface which is formed by the hub portion 10 and is configured to abut against the retention means 36 of the separable element 30. Further, as shown in **Fig. 1****,** the rotating means 17 of the hub portion 10 is a partially curved guide track which is configured to abut against the rotating means 37 of the separable element 30 so that a rotation of the separable element 30 relative to the hub portion 10 can be achieved.

Further, as illustrated in **Figs. 4****,** **6, and** 7, the retention means 36 of the separable element 30 is an abutment surface which is formed on a first end of a rib which is formed on the inside of the tubular separable element 30. The rib extends substantially parallel to the longitudinal axis 11. The rotating means 37 of the separable element 30 is an abutment surface which is formed on a second end of said rib. Hence, the retention means 36 of the separable element 30 and the rotating means 37 of the separable element 30 are integrally formed.

Moreover, the hub portion 10 has a longitudinal recess which extends parallel to the longitudinal axis 11 and through the circumferential abutment surface being the retention means 16 of the hub portion 10 (s. **Fig. 1**). Hence, in the initial position the separable element 30 is arranged in the protection element 20 such that the integrally formed retention means 36 and rotating means 37 can slide through the longitudinal recess.

Furthermore, as e.g., illustrated in **Figs. 2 and 3** the hub portion 10 comprises a first abutment surface 14 and the protection element 20 comprises a second abutment surface 24. If in the final position a pressure is applied onto the contact surface 21 such that at least one of the separated locking parts 31, 32 abuts the first abutment surface 14 and the second abutment surface 24, then the protection element 20 is blocked by said separated locking part from returning into the injection position. This situation is shown in **Fig. 6****.**

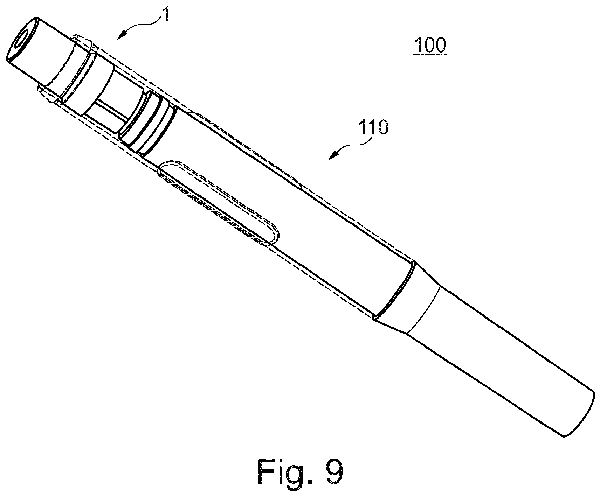

**Fig. 9** shows the medical injection device 100 according to the present disclosure which comprises the needle safety device 1 as described above. As above, the medical injection device 100 comprises a housing 110 which engages with the protection element 20 such that the protection element 20 substantially does not rotate relative to the hub portion 10.

### List of reference signs

- 1: needle safety device
- 10: elongated hub portion
- 11: longitudinal axis
- 12: cannula
- 13: tip of the cannula
- 14: first abutment surface
- 16: retention means
- 17: rotating means
- 20: tubular protection element
- 21: contact surface
- 22: opening in the contact surface
- 24: second abutment surface
- 25: protrusion
- 30: tubular separable element
- 31: first locking part
- 32: second locking part
- 35: groove
- 36: retention means
- 37: rotating means
- 40: spring element
- 100: medical injection device
- 110: housing

## Claims

1. A needle safety device (1) for a medical injection device (100), the needle safety device (1) comprising:
an elongated hub portion (10) having a longitudinal axis (11), wherein the hub portion (10) comprises a cannula (12) extending from the hub portion (10) substantially along the longitudinal axis (11);
a tubular protection element (20) being arranged to be movable along the longitudinal axis (11) relative to the hub portion (10), wherein the protection element (20) comprises a contact surface (21) for establishing pressure contact with an injection area, wherein in an initial position of the needle safety device (1) the contact surface (21) is further spaced apart from the hub portion (10) along the longitudinal axis (11) than the tip (13) of the cannula (12);
a tubular separable element (30) being removably arranged in the protection element (20) in the initial position such that the separable element (30) is movable along the longitudinal axis (11) together with the protection element (20), wherein the separable element (30) comprises at least two locking parts (31,32), and
a spring element (40) which biases the protection element (20) in a distal direction relative to the hub portion (10) such that the contact surface (21) is further spaced apart from the hub portion (10) along the longitudinal axis (11) than the tip (13) of the cannula (12) when the contact surface (21) is not in pressure contact with the injection area,
wherein the protection element (20) is configured to move into an injection position when the contact surface (21) is brought in pressure contact with the injection area, wherein in the injection position the tip (13) of the cannula (12) is further spaced apart from the hub portion (10) along the longitudinal axis (11) than the contact surface (21), wherein the separable element (30) is removed from the protection element (20) and thereby separates into the at least two locking parts (31, 32) when the contact surface (21) is released from pressure contact with the injection area, wherein the separated locking parts (31, 32) block the protection element (20) from returning into the injection position when the protection element (20) has moved to a final position, wherein in the final position the contact surface (21) is further spaced apart from the hub portion (10) along the longitudinal axis (11) than the tip (13) of the cannula (12).

2. The needle safety device (1) according to the preceding claim, wherein in the initial position the at least two locking parts (31, 32) are not materially bonded to each other.

3. The needle safety device (1) according to any one of the preceding claims, wherein in the initial position the separable element (30) is at least partially enclosed by the protection element (20) such that the at least two locking parts (31, 32) are hold together and abut against each other.

4. The needle safety device (1) according to claim 1, wherein in the initial position the at least two locking parts (31, 32) are integrally formed with each other.

5. The needle safety device (1) according to any one of the preceding claims, wherein the contact surface (21) has an opening (22) through which the tip (13) of the cannula (12) passes when the protection element (20) is transferred into the injection position.

6. The needle safety device (1) according to any one of the preceding claims, wherein the protection element (20) is configured such that it substantially does not rotate relative to the hub portion (10).

7. The needle safety device (1) according to any one of the preceding claims, wherein the protection element (20) and the separable element (30) are configured such that they allow for a rotation relative to each other, wherein optionally the separable element (30) is in rotatable sliding engagement with the protection element (20).

8. The needle safety device (1) according to any one of the preceding claims, wherein the hub portion (10) and the separable element (30) each comprise retention means (16, 36) being configured to engage with each other such that the separable element (30) is retained in a position relative to the hub portion (10) when the protection element (20) moves to the final position.

9. The needle safety device (1) according to the preceding claim, wherein the hub portion (10) and/or the separable element (30) comprise rotating means (17, 37) which rotate the separable element (30) relative to the hub portion (10) when the protection element (20) with the separable element (30) arranged therein is moved from the initial position into the injection position, wherein rotating the separable element (30) relative to the hub portion (10) leads to the retention means (16, 36) to engage with each other.

10. The needle safety device (1) according to any one of the preceding claims, wherein in the initial position the separable element (30) is removably arranged in the protection element (20) by means of a form-fit connection, wherein optionally the protection element (20) comprises a protrusion (25) and the separable element (30) comprises a groove (35), wherein in the initial position the protrusion (25) and the groove (35) are in engagement.

11. The needle safety device (1) according to any one of the preceding claims, wherein the at least two locking parts (31, 32) move radially outward as seen from the longitudinal axis (11) and thereby separate from each other when the separable element (30) is removed from the protection element (20), wherein optionally the hub portion (10) and/or the separable element (30) have separating means for moving the at least two locking parts (31, 32) radially outward.

12. The needle safety device (1) according to any one of the preceding claims, wherein the hub portion (10) comprises a first abutment surface (14) and the protection element (20) comprises a second abutment surface (24), wherein if in the final position a pressure is applied onto the contact surface (21) such that at least one of the separated locking parts (31, 32) abuts the first abutment surface (14) and the second abutment surface (24) then the protection element (20) is blocked by said separated locking part from returning into the injection position.

13. The needle safety device (1) according to any one of the preceding claims, wherein the spring element (40) abuts the hub portion (10) and the protection element (20).

14. A medical injection device (100) comprising the needle safety device (1) according to any one of the preceding claims.

15. The medical injection device (100) according to the preceding claim, wherein the medical injection device (100) comprises a housing (110) which engages with the protection element (20) such that the protection element (20) substantially does not rotate relative to the hub portion (10).
